# EUROPEAN PATENT APPLICATION

(11) **EP 4 483 800 A2**
(43) Date of publication of application: **01.01.2025**
(21) Application number: 24185300.1
(22) Date of filing: 28.06.2024
(51) Int. Cl.: A61B 5/06, A61B 5/07, A61B 5/22, A61B 5/00

(54) **DETECTION OF INTRA-BODY DEVICE SLIPS**

(30) Priority: 29.06.2023 EP 23398014
(71) Applicant: SWORD Health S.A., 4100-467 Porto (PT)
(72) Inventor: COELHO ALVES, José Carlos, 4100-467 Porto (PT); OLIVEIRA SANTOS, Pedro Henrique, 4100-467 Porto (PT); XAVIER RODRIGUES, Pedro Filipe, 4100-467 Porto (PT); HELENURM, Mark Kaius, Utah, 84020 (US); DIAS ANDRADE, João Paulo, 4100-467 Porto (PT); MOUTINHO COLUNAS, Márcio Filipe, 4100-467 Porto (PT); CARDEANO PEDROSA E MILHEIRO MAIA, Marta Maria, 4100-467 Porto (PT); FERRO BENTO, Virgílio António, 4100-467 Porto (PT)
(74) Representative: Elion IP, S.L.

(57) **Abstract**

A method comprising: processing values measured by one or more sensors of an intra-body device, and/or values of level of power of wireless data transmissions transmitted and/or received by one or more antennas of the intra-body device; establishing a slip likelihood based on the processed values, the slip likelihood being a likelihood of the intra-body device having slipped out from a cavity of a person; and determining that the intra-body device has slipped out from the cavity of the person when the slip likelihood has at least reached a predetermined level.

## Description

### TECHNICAL FIELD

The present disclosure relates to the field of intra-body devices. More particularly, but not exclusively, the present disclosure relates to the automatic detection of a slipping event corresponding to the egress of an intra-body device from a cavity of a user.

### BACKGROUND

Devices intended to be introduced into a cavity in the body of a person are referred to herein as intra-body devices. Intra-body devices such as these are typically becoming smaller in size. Housings of intra-body devices also tend to have a shape suitable for easy and gentle introduction into the cavity. Easy and gentle introduction into the cavity can further be facilitated by manufacturing such housings from softer and smoother materials. These features often also facilitate the extraction of the intra-body device from the cavity, which is useful unless the extraction occurs inadvertently or undesirably.

Some intra-body devices allow for tracking the activity of muscles of the person, including certain muscles that are difficult to monitor from external observation of the body of the person.

For example, intra-body devices are introducible into a cavity such as the vagina or the anus of the person to track pelvic floor muscles. The use of an intra-body device of this kind can enable automatic pelvic floor muscle tracking without requiring the supervision of a physical therapist or a personal trainer, because the intra-body device provides measurements that, once processed, are indicative of exercises performed by the user. For example, pressure applied to the intra-body device by muscles is indicative of the health condition of the pelvic floor and/or how the pelvic floor exercises are performed by the pelvic floor of the user.

### DESCRIPTION

It is not uncommon that, during pelvic floor exercising, the user assumes a posture in which the intra-body device slips out of the cavity without the user noticing. Depending on the sensitivity of the user in the region where the intra-body device is located, it may take longer for the user to notice that the device has slipped out. While the device is outside the cavity, the tracking of the muscles cannot be conducted, thereby increasing the risk of the user getting injured if she or he is not timely informed of possible erroneous execution of movements and exercises as prescribed by a physical therapist or a personal trainer.

Examples described herein address or alleviate the technical challenge of prompt detection of an intra-body device slipping out of the cavity of the user.

A first aspect relates to a method. In some embodiments, the method includes digitally processing values measured by one or more sensors of an intra-body device, and/or level of power values of wireless data transmissions transmitted and/or received by one or more antennas of the intra-body device. The method includes digitally establishing a slip likelihood based on the processed values, and digitally determining that the intra-body device has slipped out from the cavity of the person when the slip likelihood has at least reached a predetermined level.

The slip likelihood is a likelihood of the intra-body device having slipped out from a cavity of a person.

The values and/or level of power measured may be any one of the following: acceleration values measured by a motion sensor (that is or comprises, e.g., an accelerometer) of the intra-body device and/or angular velocity values measured by a motion sensor (that is or comprises, e.g., a gyroscope) and/or pressure values measured by a pressure sensor or a force sensor of the intra-body device and/or temperature values measured by a temperature sensor (e.g., a thermometer) of the intra-body device and/or level of power values of electromagnetic waves captured or radiated by one or more antennas of the intra-body device. The respective values and/or level of power are/is digitally processed when the slip likelihood is established based upon them.

There is typically a wide range of exercises that can be performed. Each user can perform the exercises differently in comparison with the way other users perform the exercises, the shape and, e.g., the temperature of the cavity of users are generally different, and there may be interferences and propagation losses at the premises where the user is. These phenomena and parameters can add variability to the detection of slip situations. Therefore, in some examples, to reduce false positives, the detection of slip situations is dependent upon the predetermined level, e.g., a probability threshold (such as a predetermined slip probability), a numerical threshold, among others, and/or the way the values are processed to compute the slip likelihood and/or the exercise performed by the user.

In some embodiments, the method includes controlling or adjusting a tracking procedure based on determining that the intra-body device has slipped out from the cavity. In some embodiments, the method includes controlling or adjusting operation of the intra-body device based on determining that the intra-body device has slipped out from the cavity.

In some embodiments, once it is determined that the intra-body device has slipped out from the cavity, one or more commands may be issued, such as, but without limitation, triggering a notification for informing the user, and/or pausing or stopping the muscle tracking procedure, and/or logging the slip in a register, etc.

Additionally, one or more commands may be issued prior to determining that the intra-body device has slipped out from the cavity. By way of example, when the slip likelihood is increasing and/or has reached a value below a value for determining the slipping, a notification may be triggered informing the user about a potential situation in which the intra-body may slip or may be slipping out of the cavity. By way of another example, when the slip likelihood is decreasing and/or has gone below a certain value, a notification may be triggered informing the user about the intra-body device being positioned well in the cavity, in particular when there has been a potential slippage situation. Further after issuing the one or more commands, in some embodiments, the method includes processing user input to update one or more predetermined levels such as levels for determining that the intra-body device has slipped out from the cavity or for triggering the one or more commands, thereby calibrating the levels for subsequent determinations.

The cavity, which can be either a vagina or an anus of the person, receives the intra-body device when the user is to use the intra-body device for one or more of, e.g., exercising, rehabilitation and/or pressure measuring, among others, in a supervised manner.

The method is a computer-implemented method that runs in one or more processing devices, in an isolated manner and/or in a distributed manner. That is to say, one, some or all steps may be run by a same processing device, or one or some steps may be run by one processing device and some other step or steps may be run by one or more other processing devices or even be run in distributed manner, which means that several processing devices cooperate to run one or more steps. In this sense, the steps are digitally run. The processing device or devices may be included in the intra-body device itself, and/or be external to the intra-body device, for example a processing device such as, but without limitation, a mobile phone, a tablet, a personal computer, a server, a field-programmable gate array, and/or an application specific integrated circuit, etc., and even be remote from the intra-body device.

In some embodiments, establishing the slip likelihood comprises computing the slip likelihood based on at least one probability of existence of slippage.

The processed values allow for computing of one or more probabilities of the intra-body having slipped out from the cavity. The processed values are indicative of slip conditions and represent potential slippage of the device. In this sense, one or more probabilities of the at least one probability of existence of slippage can relate to processed values of a sensor of the intra-body device.

In some embodiments, establishing the slip likelihood comprises associating at least one predetermined relationship between some or all processed values and the at least one probability of existence of slippage.

The at least one probability of existence of slippage may be computed by way of one or more predetermined relationships (e.g., at least one equation, at least one condition to be met, etc.) that relate the numerical values of the processed values with greater or lower probability of slippage.

For example, a way of computing the slip likelihood in some cases is by establishing how the probability of existence of slippage increases and/or decreases based on how the processed values evolve over time. A rate of increase of the probability may be defined, for example, as increasing the probability as the processed values increase or as the processed values decrease; a rate of decrease of the probability may be defined in a similar fashion. By way of a non-limiting example, acceleration values with a magnitude that is above a certain level for a period of time or with an increasing trend may increase the probability of a slip situation; and/or acceleration values with a magnitude that is below a certain level for a period of time or with a decreasing trend may reduce the probability of existence of slippage.

For example, the presence of values outside a predetermined range, either intermittently or continuously, or values that are considerably different from other values part of the same subset of values provide ways of computing the slip likelihood in some cases. By way of a non-limiting example, angular velocity values may be integrated to detect tilts that are not within a predetermined range or tilts that can be considered outliers with respect to other tilts may increase the probability of existence of slippage. Conversely, values within the predetermined range and/or without significant variation may reduce the probability of existence of slippage.

In some embodiments, establishing the slip likelihood comprises aggregating some or all probabilities of the at least one probability of existence of slippage.

Each set of processed values provides a respective probability of existence of slippage, or even multiple respective probabilities of slip situation when a single set of values may be processed in different ways to derive multiple probabilities of slip situation. For example, angular velocity values may be used to provide a first probability of existence of slippage by evaluating the angular velocity, and the same values be used to provide a second probability of existence of slippage by evaluating an integral of the angular velocity values.

In some embodiments, the processed values for establishing the slip likelihood at least comprise first and second processed values.

The first processed values and the second processed values may relate, in some embodiments, to measurements of a same sensor, e.g., the same pressure sensor or the same pressure sensors, or a sensor of the same type, e.g. the first processed values relate to measurements of a motion sensor or a first accelerometer thereof and the second processed values relate to measurements of a second accelerometer of the same motion sensor or an accelerometer of another motion sensor.

In some embodiments, the slip likelihood is established to a first predetermined level when one of first and second conditions is met, and to a second predetermined level when the first and second conditions are both met.

The detection of slip situations is related to the predetermined level and/or how the slip likelihood is computed, yet both can be associated with a number of conditions to be met to determine that a slip has occurred. In this respect, the detection of slip situation optionally also depends upon which specific conditions are met. Each predetermined level relates to a specific probability of slippage of the intra-body device; therefore, each greater level is indicative of a greater probability of slippage.

Each of the first and second conditions (and third and further conditions, if any) is associated with at least some processed values. The first condition may for example be associated with some first processed values, and the second condition may for example be associated with some second processed values, or vice versa. The conditions may be met when a specific event occurs as revealed by the corresponding processed values and/or when an associated probability of existence of slippage (if any) reaches a certain value (e.g., 50%, and/or 80%, and/or 100%, etc.).

When multiple processed values are evaluated, the sets of processed values (e.g., the first and second processed values, and further processed values if any) may be values taken within a common predetermined time window. The determination of the slip situation can be more accurate when it is made with an evaluation of values occurring for a same event or set of events (e.g., a repetition of a movement, performance of an exercise, among others).

The common predetermined time window is sufficiently long to enable detection of potential slip situations occurring progressively or sequentially (e.g., one after another but not necessarily immediately one after another), but is not unnecessarily long, otherwise it may potentially detect two unrelated potential slip situations as being part of the same slip of the intra-body device. For example, but without limitation, the time window may last 1 second, and/or 2 seconds, and/or 4 seconds, and/or 6 seconds, etc. In some cases, the time window is less than 15 seconds, and/or 10 seconds and/or 5 seconds. The length of the time window might also be set depending on the time it takes the user to perform one or several repetitions of a given exercise since a slip will usually occur in one repetition or over the course of, e.g., two to five repetitions. Thus, upon processing the activity of the muscles of the user and determining the performance of repetitions, the method can include, in some embodiments, adjusting the length of the time window based on the determination made. The common predetermined time window can be a sliding time window so that it moves until a first slip situation is detected.

In some embodiments, determining that the intra-body device has slipped out from the cavity of the person is when the slip likelihood has at least reached the second predetermined level. The second predetermined level is greater than the first predetermined level.

The slip likelihood increases as conditions are met because each condition relates to a potential slip situation. When the slip likelihood reaches a certain level, it is determined that the intra-body device has slipped out from the cavity.

For the slip likelihood to increase to or above the second predetermined level or predetermined levels greater than the second predetermined level, several processed values taken during a common predetermined time window should be indicators of a potential slip situation. In some embodiments, a probability of the at least one probability of existence of slippage relates to processed acceleration values. Said probability increases when at least some values of the processed acceleration values have, in at least one predetermined acceleration direction, an increasing magnitude or the magnitude exceeds a predetermined acceleration threshold, the magnitude thereby being outside a predetermined acceleration range. Said probability may decrease or remain unchanged otherwise.

In some embodiments, in addition to or instead of said probability, the first condition or the second condition (or, in some other embodiments, a third or subsequent condition) requires that at least some processed acceleration values have a magnitude that exceeds a predetermined acceleration threshold in at least one predetermined acceleration direction.

Accelerations can be indicators of potential slip situations because accelerations are prone to unexpected variations when the intra-body device slips from the cavity. Typically, when the device is outside the body, the device and, thus, the motion sensor have more freedom of movement as they are not constrained by the cavity, in which case the acceleration values are typically higher than when constrained by the cavity. Of course, if in addition to the greater movement freedom, the device even hits a surface such as the ground, the hit resulting from the fall will result in a peak acceleration.

In some embodiments, the at least one predetermined acceleration direction includes a direction defined by a segment from the first end of the intra-body device to a second end of the intra-body device. The first end is opposite the second end, and the first end is the end of the intra-body device through which the intra-body device is introduced in the cavity of the person.

A set of meaningful accelerations to be monitored for detecting potential slip situations are those corresponding to the acceleration directions between opposite ends of the intra-body device, and such that the directions go from the end first introduced in the cavity to the other end of the device that, in many cases, is outside the cavity. When there are slips, the accelerations go in those directions since they are the egress directions.

In some embodiments, a probability of the at least one probability of existence of slippage relates to processed pressure values. Said probability increases when at least some (first or second) values of the processed pressure values have an increasing magnitude or a magnitude that exceeds a (first or second) predetermined pressure threshold for at least one frequency above a first predetermined frequency threshold, the magnitude thereby being outside a predetermined pressure range. Said probability may decrease or remain unchanged otherwise.

In some embodiments, in addition to or instead of said probability, the first condition or the second condition (or, in some other embodiments, a third or subsequent condition) requires that at least some (first or second) values of processed pressure values have a magnitude that exceeds a (first or second) predetermined pressure threshold for at least one frequency above a first predetermined frequency threshold.

The frequency spectrum for the processed pressure values is obtained so that frequency patterns can be detected. Multiple frequency spectra may be obtained for several sets of processed pressure values within the common predetermined time window to be able to compare at which frequencies there are pressures and how intense they are in comparison with other pressures within the same time window. Any suitable technique for obtaining the frequency spectra is possible, for example, the discrete Fourier transform, the fast Fourier transform, etc.

When a user performs deliberate contractions or relaxations, pressures at low frequencies (e.g., 0.5 Hz, 1.0 Hz) are usually present. By contrast, when the user performs non-deliberate movements, the pressures generally occur at higher frequencies. The first predetermined frequency threshold can be set so as to not encompass frequencies associated with deliberate contractions or relaxations, thus the value of such threshold may be of, e.g., 3 Hz, 3.5 Hz, etc.

In some embodiments, the established slip likelihood is reset to a predetermined baseline level when the established slip likelihood has not reached the predetermined level during a predetermined holding time and/or when the exercise to be performed by the user changes from a first predetermined exercise to a second predetermined exercise.

In some embodiments, the established slip likelihood is maintained at a level greater than a predetermined baseline level during a predetermined holding time before being established to the predetermined baseline level; and the first predetermined level is greater than the predetermined baseline level.

The slip likelihood is to be reset after some time has elapsed and no slip has been determined as having occurred. This way, the slip likelihood is not influenced by potential slip situations having occurred sometime before.

The predetermined baseline level may be set to any value convenient for establishing the initial slip likelihood. The predetermined baseline level is indicative of the intra-body device being within the cavity (e.g., there is no slip situation). For example, the value may be set to zero or some other value.

When the slip likelihood is established based on a number of predetermined levels, the first and second predetermined levels are greater than the predetermined baseline level, at least in absolute terms, since the slip likelihood and the levels may be set up with negative sign(s); or even be lower than the predetermined baseline level if the baseline is set as a maximum level as an indicator of insertion likelihood and the predetermined levels being lower the lower the likelihood that the intra-body device is inserted in the cavity. In any of these cases, the second predetermined level would be lower than the first predetermined level: the second predetermined level is more negative than the first predetermined level, or both levels are positive but the second predetermined level is lower than the first predetermined level.

In some embodiments, the predetermined holding time is equal to a length of the common predetermined time window. This forces all slip situations that increase the slip likelihood to occur within the common predetermined time window, and it causes the determination of there being a slip if the slip likelihood has increased high enough (e.g., it has at least reached the predetermined level or it has at least reached a particular predetermined level) within said time window.

In some embodiments, a probability of the at least one probability of existence of slippage relates to processed pressure values. Said probability increases when at least some (first or second) values of the processed pressure values have a decreasing magnitude or a magnitude that is below a (first or second) predetermined pressure threshold for at least one frequency above a first predetermined frequency threshold, the magnitude thereby being outside a predetermined pressure range. Said probability may decrease or remain unchanged otherwise.

In some embodiments, in addition to or instead of said probability, the first condition or the second condition (or, in some other embodiments, a third or subsequent condition) requires that at least some (first or second values) of processed pressure values have a magnitude that is below a (first or second) predetermined pressure threshold for at least one frequency above a (first or second) predetermined frequency threshold. In some embodiments, when there are at least first and second predetermined frequency thresholds, this predetermined frequency threshold is lower than the predetermined frequency threshold corresponding to the probability that increases for high pressure values or the condition that requires processed pressure values to have a high pressure (above a predetermined pressure threshold) for at least one frequency above said other threshold.

Following a slip, typically the pressure values stabilize and are of low intensity and frequency because no muscles are exerting force on the intra-body device. This can also be an indicator of a potential slip situation. By analyzing whether the processed pressure values behave in this manner for frequencies below a predetermined frequency threshold, a potential slip situation may be detected and have a corresponding computed probability or condition associated therewith that will establish a different level for the slip likelihood.

In some embodiments, processed pressure values (e.g., the some processed pressure values) associated with the computed probability or condition related to the second predetermined frequency threshold (corresponding to low pressure values for certain frequencies) are measured after processed pressure values (e.g., the some processed pressure values) associated with the condition related to the first predetermined frequency threshold (corresponding to high pressure values for certain frequencies).

As aforesaid, the stable and low frequency pressure values occurring after a slip tend to occur after there have been pressure values at mid-range frequencies caused by non-deliberate movements.

In some embodiments, a probability of the at least one probability of existence of slippage relates to processed acceleration values. Said probability increases when there is variation in acceleration vector exceeding a predetermined acceleration variation threshold in a predetermined period of time, the variation thereby being outside a predetermined acceleration variation range, and/or the processed acceleration values have an increasing magnitude in the predetermined period of time. Said probability may decrease or remain unchanged otherwise.

In some embodiments, in addition to or instead of said probability, the first condition or the second condition (or, in some other embodiments, a third or subsequent condition) requires that at least some processed acceleration values are indicative of a variation in acceleration vector exceeding a predetermined acceleration variation threshold in a predetermined period of time, and/or processed acceleration values have a magnitude that has increased more than a predetermined acceleration increase threshold in the predetermined period of time.

In slip situations, there is usually a high rate of variation in the angle of acceleration direction (e.g., acceleration vector) of the acceleration measurements of the device, and/or a magnitude of the acceleration measurements that increases monotonically beyond the predetermined acceleration increase threshold. This is due to the intra-body device moving in a way that is inconsistent with the functional exercises being performed.

In some embodiments, a probability of the at least one probability of existence of slippage relates to processed acceleration values. Said probability increases when at least some values of the processed acceleration values have an increasing magnitude or a magnitude that exceeds a predetermined acceleration threshold for at least one frequency above a predetermined acceleration frequency threshold, the magnitude thereby being outside a predetermined acceleration range. Said probability may decrease or remain unchanged otherwise.

In some embodiments, in addition to or instead of said probability, the first condition or the second condition (or, in some other embodiments, a third or subsequent condition) requires that at least some processed acceleration values are indicative of an increasing magnitude over time (e.g., a predetermined period of time) or a magnitude that exceeds a predetermined acceleration threshold for at least one frequency above a predetermined acceleration frequency threshold.

The processing of acceleration values may be such that it provides an analysis of acceleration frequencies, which indicates whether there are slow acceleration changes (e.g., relatively low frequencies, for example of 1 Hz or less) and/or fast acceleration changes (e.g., relatively high frequencies, for example of 3 Hz or more). Fast acceleration changes may be particularly representative of a potential slip situation because they are not expected in pelvic floor exercises, hence higher fast acceleration changes increase the associated probability more rapidly.

Accelerations in a predetermined axis are, in some cases, ignored in the calculation of the probability of existence of slippage or the meeting of one or more conditions depending on the exercise to be performed by the user. The predetermined axis is set to the axis of acceleration expected for the particular exercise, therefore an existing acceleration or a variating acceleration in such axis is representative of a normal behavior of the intra-body device.

In some embodiments, a probability of the at least one probability of existence of slippage relates to processed basal pressure values. Said probability increases when at least some values of the processed basal pressure values have a greater (in absolute value) variation in decreasing magnitude or a variation (in absolute value) in decreasing magnitude that is above predetermined basal pressure variation threshold, the variation in decreasing magnitude thereby being outside a predetermined basal pressure variation range. Said probability may decrease or remain unchanged otherwise.

In some embodiments, in addition to or instead of said probability, the first condition or the second condition (or, in some other embodiments, a third or subsequent condition) requires that at least some values of processed basal pressure values have a greater (in absolute value) variation in decreasing magnitude or a variation (in absolute value) in decreasing magnitude that is above predetermined basal pressure variation threshold.

Basal pressure decreases are representative of the placement of the intra-body device within the cavity or outside thereof. Particularly, the decreases in basal pressure may be related to whether the intra-body device is within the cavity, which is generally the case when there is no decrease in basal pressure beyond a certain value, and outside the cavity otherwise.

For instance, in particular exercises such as, e.g., a lying on the back exercise, a greater basal pressure is indicative of a probable correct placement of the intra-body device, and a lower basal pressure is indicative of a potential or probable slippage of the intra-body device. As the magnitude of the pressure values decreases, the probability of existence of slippage increases.

In some embodiments, a probability of the at least one probability of existence of slippage relates to processed angular velocity values. Said probability increases when at least some values of the processed angular velocity values have an increasing magnitude over time (e.g., a predetermined period of time) or a magnitude that exceeds a predetermined angular velocity threshold, the magnitude thereby being outside a predetermined angular velocity range. Said probability may decrease or remain unchanged otherwise.

In some embodiments, in addition to or instead of said probability, the first condition or the second condition (or, in some other embodiments, a third or subsequent condition) requires that at least some values of processed angular velocity values have a magnitude that exceeds a predetermined angular velocity threshold.

Angular velocities tend to be reduced or even zero for many pelvic floor exercises. The existence of angular velocities may result in an increase in probability of existence of slippage. An increase in probability or meeting a condition is also possible when the angular velocities are not within an acceptable range of angular velocities that represents the expectable angular velocities while performing a certain exercise.

As explained before in relation to accelerations, angular velocities in a predetermined axis are, in some cases, ignored in the calculation of the probability of existence of slippage or the meeting of one or more conditions depending on the exercise to be performed by the user. This is so because particular exercises require rotations that produce angular velocities in a particular direction.

In some embodiments, a probability of the at least one probability of existence of slippage relates to processed angular velocity values. Said probability increases when an integration of at least some values of the processed angular velocity values exceeds a predetermined integrated angular velocity threshold, the integration thereby being outside a predetermined integrated angular velocity range. Said probability may decrease or remain unchanged otherwise.

In some embodiments, in addition to or instead of said probability, the first condition or the second condition (or, in some other embodiments, a third or subsequent condition) requires that an integration of at least some values of the processed angular velocity values exceeds a predetermined integrated angular velocity threshold.

The integration of angular velocities results in the provision of tilts, which represent how the motion sensor measuring the angular velocities is tilted or oriented with respect to an initial tilt or orientation. The probability of existence of slippage may increase as the tilt is larger unless the exercise performed by the user involves certain tilt values. In some embodiments, a probability of the at least one probability of existence of slippage relates to processed values of electromagnetic waves. Said probability increases when first electromagnetic waves corresponding to wireless data transmissions captured and/or radiated by the intra-body device are indicative of a level of power at a device capturing the first electromagnetic waves that is greater than the level of power of second electromagnetic waves captured by the device capturing the first and second electromagnetic waves. Said probability may decrease or remain unchanged otherwise. Optionally, the increase in said probability may occur only when the difference in level of power exceeds a predetermined power level threshold, the difference thereby being outside a predetermined difference range.

In some embodiments, in addition to or instead of said probability, the first condition or the second condition (or, in some other embodiments, a third or subsequent condition) requires that: first electromagnetic waves corresponding to wireless data transmissions captured and/or radiated by the intra-body device are indicative of a level of power at a device capturing the first electromagnetic waves that is greater than the level of power of second electromagnetic waves captured by the device capturing the first and second electromagnetic waves by more than a predetermined power level threshold. In these cases, the first electromagnetic waves are captured after the second electromagnetic waves.

When the intra-body device is within the cavity, the wireless communications between the intra-body device and any other device remote therefrom, for example a computing device that registers measurements or processed values of the intra-body device to track the pelvic floor muscle activity and generate a pelvic floor muscle activity sequence, is usually worse in signal than when the intra-body device is not within the cavity. The presence of the intra-body device within the cavity may alter the communications owing to any one of the following phenomena: greater propagation losses, greater interferences, and/or detuning of the antenna(s) of the intra-body device.

If the level of power of captured waves increases relative to the level of power of previous captured waves, a slip situation is potentially detected because the change is a hint of possible egress of the intra-body device.

In some embodiments, the level of power of the first and second electromagnetic waves is established by a level of power with which the intra-body device or the device at the receiving end of the wireless data transmissions received the waves or a strength of a signal thereof, such as received signal strength indicators, RSSI, that is subsequently included in data packets within the wireless data transmissions of the first and second electromagnetic waves.

The probability and/or condition related to the level of power of the electromagnetic waves can be set with reference to, e.g., RSSI values within the data packets transmitted. In this sense, the intra-body device may include such values in the data packets after having measured the power with which the intra-body device receives wireless communications from the other device, e.g., a computing device with which the intra-body device communicates, or vice versa.

In some embodiments, a probability of the at least one probability of existence of slippage relates to processed temperature values. Said probability increases when at least some values of processed temperature values are indicative of a certain rate of temperature invariancy (i.e., constancy), of a decrease in temperature, or a slow rate of temperature increase over time (e.g., a predetermined period of time). In some cases, the rate of temperature invariancy or rate of temperature decrease or a slow rate of temperature increase over time is such that it is greater, in magnitude, than a predetermined temperature invariancy or decrease or slow increase rate, the rate of temperature invariancy or decrease or slow rate of temperature increase thereby being outside a predetermined temperature rate change range. Said probability may decrease or remain unchanged otherwise. In some embodiments, said probability at least reaches the predetermined level when the at least some values of processed temperature values are indicative of a certain rate of temperature invariancy, rate of temperature decrease or slow rate of temperature increase over time.

In some embodiments, in addition to or instead of said probability, the first condition or the second condition (or, in some other embodiments, a third or subsequent condition) requires that at least some values of processed temperature values are indicative of a rate of temperature invariancy or rate of temperature decrease or a slow rate of temperature increase over time (e.g., a predetermined period of time) that, in magnitude, is greater than a predetermined temperature invariancy or decrease or slow increase rate.

Temperature measurements are an indicator of potential slip situations because the temperature in the cavity is generally higher than ambient temperature. Therefore, when there is a decrease in temperature, or slow increase in temperature, or a substantially unchanged temperature, in a given period of time, e.g., 1 second, and/or 2 seconds, and/or 10 seconds, etc., the device may have slipped out from the cavity. By way of example, if there is a rate of temperature decrease of 0.3 degrees Celsius or more per 2 seconds, an increase in probability may be triggered or a condition for increasing the slip likelihood could be met. Likewise, when the temperature change is flattening, such as when there is almost no change in temperature in a period of time of, e.g., 15 seconds, an increase in probability may be triggered or a condition for increasing the slip likelihood could be met.

The period of time considered for said rate may or may not be the window considered for evaluating the slip likelihood, hence the period of time for the rate may even be longer or shorter than the time window.

In some embodiments, establishing the slip likelihood comprises weighing one or more probabilities of the at least one probability of existence of slippage and/or adjusting a maximum limit for the one or more probabilities based on an exercise to be performed by a user and/or based on the user to perform the exercise.

In some embodiments, establishing the slip likelihood comprises adjusting one or more predetermined levels or thresholds for conditions to be met based on an exercise to be performed by a user.

Different exercises involve different postures and efforts by the user. These, in turn, condition measurements of sensor or sensors of the intra-body device. Therefore, by modifying the weight and/or maximum value(s) of the one or more probabilities and/or thresholds associated with conditions, the determination of the slippage may be improved since it is tailored to the situation that the user is or will be in. Data of the user to perform the exercise such as, e.g., muscular mass, existing injuries, fit condition, etc., may also be used to adjust weights and/or maximum limits to better adapt the determinations made to the status of the user.

By way of example, in a lengthening exercise or in a pressure basal calibration where very low pressure values are measured, reductions in processed basal pressure values have a small weight or even a weight of zero so that it does increase the slip likelihood little or does not increase it all.

By way of another example, in a lying side exercise, angular velocities in respect of one axis have a small weight or even a weight of zero so that it does increase the slip likelihood little or does not increase it all.

By way of further examples, in an exercise in which the user does not have the hip on the floor, the weight of acceleration values may be made greater whereas, in an exercise in which the user is stationary and contracts and relaxes the pelvic floor, the weight of rotation values may be greater as well; in both cases, the existence of such values is potentially representative of a slip situation and so the greater weight increases the probability of determining a potential slip.

In some embodiments, the method further includes, when it is determined that the intra-body device has slipped out from the cavity of the person, halting tracking of pelvic floor muscles of the person based on the processed pressure values. This is an example of controlling or adjusting a tracking procedure based on determining that the intra-body device has slipped out from the cavity.

Pressure values of at least one pressure or force sensor of the intra-body device are used for the tracking of the activity of the pelvic floor muscles, so the at least one pressure or force sensor may double as a potential slip detector when such sensor is also used for detecting slippage. The tracking of the pelvic floor muscles, which is carried out by processing the different processed values so as to generate a pelvic floor muscle activity sequence indicative of how the muscles have behaved, can be halted when it is determined that there is a slip, thereby not altering the registered activity of the user with meaningless information while the intra-body device is outside the body. The halting can be temporary, in which case it is paused at least during the time that the intra-body device is outside the body, or permanent, in which case the tracking session is ended until a new tracking session is started.

In some embodiments, the slip likelihood is established to a predetermined level greater than the second predetermined level when a third or a subsequent condition is met, and the slip likelihood is already at a level equal to or greater than the second predetermined level.

In some embodiments, the determination of the intra-body device having slipped out from the cavity is made when the slip likelihood has at least reached a level greater than the second predetermined level.

When determining the existence of slippage with multiple predetermined levels, to reduce the number of false positives, more than two slip situations may have to be detected prior to determining that the intra-body device has slipped out from the cavity. To that end, more predetermined levels greater than the second predetermined level may be set up, and the slip likelihood be made to progress over the different levels based on the conditions met.

Although it has been described that, in some embodiments, the slip likelihood is established to the first and second predetermined levels (and, optionally, further predetermined levels beyond the second predetermined level) sequentially (i.e., the slip likelihood must be at the first predetermined level for it to be established to the second predetermined level, or be at the second predetermined level for it to be established to the subsequent predetermined level), it will be noted that it is also possible to conduct the establishment of level of the slip likelihood at once upon simultaneous meeting of the different criteria set up. The latter behavior, however, may be more demanding in terms of processing power, and more difficult to take place since not all slip situations occur at once.

In some embodiments, for digitally establishing the slip likelihood to the predetermined level greater than the second predetermined level, at least two of the following criteria are met: processed acceleration values are indicative of the variation in acceleration vector exceeding the predetermined acceleration angular variation in the predetermined period of time; processed acceleration values have a magnitude that has increased more than the predetermined acceleration increase threshold in the predetermined period of time; and the first electromagnetic waves are indicative of the level of power greater than the level of power of the second electromagnetic waves by more than the predetermined power level threshold.

In some embodiments, the method further includes, when it is determined that the intra-body device has slipped out from the cavity of the person, digitally commanding to provide or providing one or more user perceptible signals indicative of the intra-body device having slipped out from the cavity. For example, the person can automatically be presented with a notification (e.g., via the intra-body device or a computing device connected to the intra-body device) that the intra-body device has slipped out. In some embodiments, the method further includes, prior to determining that the intra-body device has slipped out from the cavity of the person, digitally commanding to provide or providing one or more user perceptible signals indicative of the intra-body device potentially slipping out from the cavity. For example, the person can automatically be presented with a notification (e.g., via the intra-body device or a computing device connected to the intra-body device) that the intra-body device may possibly slip out.

Accordingly, in some embodiments, the method notifies the user whenever the slip has occurred and/or before that occurs so that the user may take corrective action, such as rearranging the intra-body device within the cavity, and/or stopping the exercising, etc. These are further examples of controlling or adjusting a tracking procedure based on determining that the intra-body device has slipped out from the cavity (or may potentially slip out).

In some embodiments, the method further includes: repeating the digitally processing, the digitally establishing, and the digitally determining steps a number of times, each time for a different common predetermined time window; and digitally reestablishing the digital determination made when, subsequent to a determination made, the slip likelihood cannot be established to the predetermined level, or cannot be established to the first predetermined level based on the first condition.

The monitoring of the slip likelihood by means of at least one probability of existence of slippage and/or the existence of at least one slip condition of the intra-body device can be conducted sequentially in a continuous manner. The exercising session is monitored during its whole duration. Whenever it is determined that there has been a slip, that determination is reset when the aggregate of the at least one probability is below a given threshold and/or none of the first and second conditions is met.

In some embodiments, the method further includes, when the slip likelihood is below the predetermined level, digitally tracking pelvic floor muscles to establish how muscles of a pelvic floor of the user are contracting or elongating based on the processed pressure values. In some embodiments, digitally tracking pelvic floor muscles takes place when the slip likelihood is in the first predetermined level or a predetermined baseline level or, optionally, the second predetermined level.

As long as no slip has been determined to have occurred, the pelvic floor muscle tracking is conducted so as to monitor the activity of the pelvic floor muscles of the user so that the exercises of the user can be automatically supervised.

In some embodiments, digitally establishing the slip likelihood to the different predetermined levels includes setting a slip likelihood parameter to one predetermined level or another, or triggering a respective slip likelihood flag.

The slip likelihood can be tracked in a number of ways, including updating the value of, e.g., a digital variable, and/or with an array of flags that have their value changed depending on the conditions being met.

A second aspect relates to a computing device or system. The computing device or system includes at least one processor; and at least one memory. Further, the at least one memory is configured, together with the at least one processor, to cause the computing device or system to carry out the steps of a method as described in the first aspect. Thus, in some embodiments, the computing device or system is inter alia configured to process values measured by one or more sensors of an intra-body device, and/or level of power values of wireless data transmissions transmitted and/or received by one or more antennas of the intra-body device. The computing device or system may establish a slip likelihood based on the processed values; and determine that the intra-body device has slipped out from the cavity of the person when the slip likelihood has at least reached a predetermined level (e.g., a probability threshold, or a numerical threshold).

A third aspect relates to a computing device or system comprising means or modules for carrying out the steps of a method as described in the first aspect.

The computing device or system may be or form part of a pelvic floor muscle tracking device or system.

In some embodiments, the computing device or system of the second and third aspects further includes the intra-body device. The intra-body device includes at least one sensor. The at least one sensor includes a pressure or force sensor.

In some embodiments, the at least one sensor further includes at least one motion sensor and/or at least one temperature sensor. For example, the at least one sensor further includes a gyroscope and an accelerometer.

A fourth aspect relates to an intra-body device that includes at least one pressure or force sensor. The intra-body device further includes, in some embodiments, at least one motion sensor and/or at least one temperature sensor. The intra-body device further includes a computing device or system that, in some embodiments, is as described in the second aspect or the third aspect.

The at least one pressure or force sensor is capable of measuring the activity of the muscles of the pelvic floor muscles so that supervision of the exercising or rehabilitation thereof is made possible.

A fifth aspect relates to a computer program comprising instructions which, when the program is executed by at least one computing device, cause the at least one computing device to carry out the steps of a method as described in the first aspect.

A sixth aspect relates to a computer-readable non-transitory storage medium comprising instructions which, when executed by at least one computing device, cause the at least one computing device to carry out the steps of a method as described in the first aspect.

A seventh aspect relates to a data carrier signal carrying a computer program as described in the fifth aspect.

### BRIEF DESCRIPTION OF THE DRAWINGS

To complete the description and in order to provide for a better understanding of the disclosure, a set of drawings is provided. Said drawings form an integral part of the description and illustrate embodiments of the disclosure, which should not be interpreted as restricting the scope of the disclosure, but just as examples of how the disclosure can be carried out. The drawings comprise the following figures:
Figure 1 is a diagram illustrating an intra-body device according to some embodiments.
Figure 2 is a diagram illustrating a pelvic floor muscle tracking system according to some embodiments.
Figure 3 is a graph illustrating a time evolution of slip likelihood, according to some embodiments.
Figure 4 is a graph illustrating a time evolution of slip likelihood, according to some embodiments.
Figure 5 is a graph illustrating a time evolution of slip likelihood, according to some embodiments.
Figure 6 is a graph illustrating a potential slip situation based on RSSI values, according to some embodiments.
Figure 7 is a graph illustrating a potential slip situation based on pressure values and frequency spectra, according to some embodiments.

### DETAILED DESCRIPTION

Examples in the present disclosure can provide one or more solutions in the field of intra-body devices, such as intra-vaginal or intra-anal devices, to problems such as the inadvertent slippage of these devices from the user's body cavity during use. This slippage can interrupt the tracking or monitoring process, leading to inaccurate data collection and potentially compromising the effectiveness of an exercise or therapy session. As a result, the intra-body device's technical performance and/or utility is degraded.

Examples described herein provide systems, devices, and/or methods to address or alleviate this problem by establishing a slip likelihood, monitoring this likelihood, and identifying when it reaches a predetermined level, thereby enabling a system or device to detect and react to a slippage event. For example, this allows for faster immediate corrective action to resume accurate monitoring and data collection. As another example, this allows the system to halt tracking (e.g., to cease using certain measurements) until the slippage event has been addressed, thereby ensuring that incorrect or inaccurate data are disregarded.

A further problem is the lack of a real-time or near real-time feedback system to alert a user of slippage events. Delays in addressing such events can result in users continuing their exercises incorrectly and/or not being able to obtain accurately tracking outputs, thereby negating the benefits of the exercises and/or increasing the risk of injury. Examples described herein provide for rapid detection of slippage and the triggering of a notification to the user, informing them of the slippage. Such real-time feedback allows the user to adjust the device or their activity.

Technical challenges can also arise due to the variability from one user to another as a result of individual differences in anatomy, the variability of the internal environment (such as muscle tone and cavity shape), and/or the presence of external interferences or propagation losses at the location of use. These factors can affect the intra-body device's ability to stay in place and accurately measure and track intended metrics. Examples in the present disclosure better address or adapt to such variability by tailoring computations of slip likelihood based on specific exercises and/or individual user characteristics. Such technical solutions can enhance the overall effectiveness of intra-body devices.

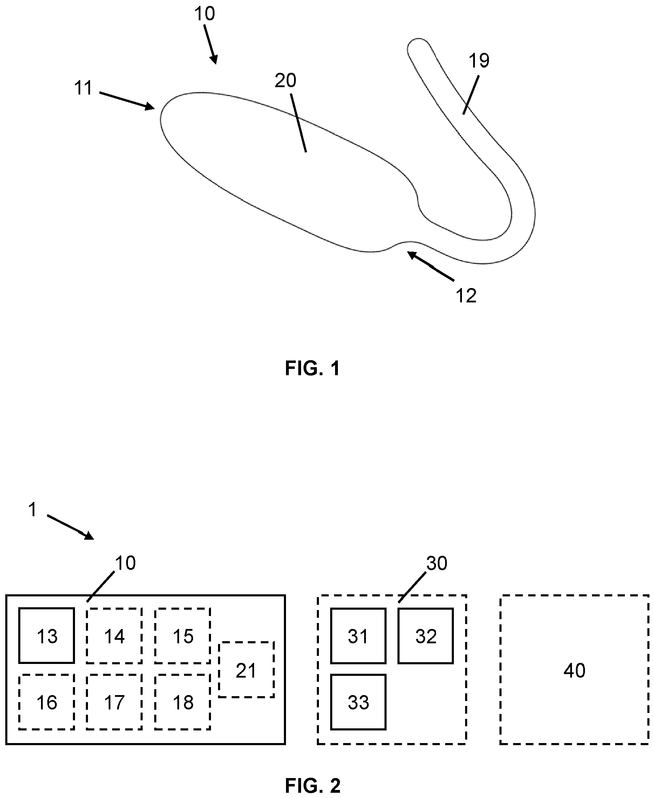

Figure 1 is a diagram illustrating an intra-body device 10 according to some embodiments. The intra-body device 10 of Figure 1 can function as an intra-vaginal device and/or an intra-anal device.

The intra-body device 10, has, in these embodiments, a main body 20, which in some examples spans between a first end 11 (e.g., an introduction end) and a second end 12 (e.g., an extraction end), and a protruding member 19 to facilitate extraction of the device 10 when inserted in a cavity. The intra-body device 10 includes at least one sensor, such as, e.g., a motion sensor, a pressure sensor, a force sensor, and/or a temperature sensor.

In some examples, the intra-body device 10 also includes a wireless communications module and at least one memory unit (not illustrated in Figure 1 since they would typically be located inside of the intra-body device 10). The at least one memory unit can store measurements of the at least one sensor of the device 10 for processing and/or transmission, and it can also store computer program code with instructions in case the intra-body device 10 conducts digital processing of the measurements. Other possible components of an intra-body device are described with reference to Figure 2 below.

Figure 2 is a diagram illustrating a pelvic floor muscle tracking system 1 according to some embodiments.

The pelvic floor muscle tracking system 1 at least includes an intra-body device 10 (such as, for example, the device 10 shown in Figure 1). The intra-body device 10 includes at least one first sensor 13 and may further include (as shown with dashed borders) a second sensor 14 and/or a third sensor 15 and/or further sensors. The intra-body device 10 also includes, in some embodiments, a data communications module 16, such as a wireless communications module. The intra-body device 10 also includes, in some embodiments, at least one memory 17 and at least one processor 18 for processing measurements or other data of the at least one sensor and/or the data communications module 16.

The pelvic floor muscle tracking system 1 also includes, in some embodiments, at least one computing or data processing device 30 as shown with dashed borders in Figure 2. The at least one computing or data processing device 30 includes at least one processor 31, at least one memory 32, and a data communication module 33, such as a wireless communications module for providing data to and/or receiving data from the intra-body device 10. The at least one computing or data processing device 30 may conduct processing of measurements provided by the at least one sensor of the intra-body device 10. The at least one computing or data processing device 30 may also process other values, such as power levels of wireless transmissions received from the intra-body device 10.

Additionally or alternatively, the pelvic floor muscle tracking system 1 includes, in some embodiments, at least one apparatus 40 for, e.g., notifying or informing users about certain information or events. The at least one apparatus 40 is or comprises, e.g., a sound-emitting device and/or a vibrator and/or a light-emitting device and/or a display. It may also include a data communications module such as a wireless communications module. The at least one apparatus 40 may be part of the intra-body device 10, part of the computing or data processing device 30, or be a separate apparatus.

The data communications modules 16, 33 may be adapted to allow wireless data exchange via, e.g., a local area network, cellular network, or the like, and/or to allow wired data exchange. In some examples, the intra-body device 10 includes at least one antenna 21 for allowing transmitting and/or receiving of wireless data transmissions to/from the computing or data processing device 30 and/or apparatus 40. The antenna 21 can be part of the data communications module 16 or separate from, but communicatively coupled to, the data communications module 16.

In some examples, the pelvic floor muscle tracking system 1 allows for the automated tracking of pelvic floor muscles or exercises. For example, the computing device 30 receives measurements from the intra-body device 10 that, once processed, are indicative of exercises performed by the user while the intra-body device 10 is inserted in a cavity (e.g., the vagina or anus). In this way, the pelvic floor muscle tracking system 1 can determine whether exercises are being performed and/or how well the user is performing them.

In some examples, the apparatus 40 and/or computing device 30 provides a user interface that provides the user with, for example, instructions or feedback with respect to positions, movements, or exercises to be performed (e.g., in the context of pelvic floor therapy). In some examples, the pelvic floor muscle tracking system 1 (e.g., via the apparatus 30 or computing device 40) automatically instructs the user to insert the intra-body device 10 and/or perform certain exercises according to a tracking procedure. For example, the user can be presented with a suitable visual and/or audible notification. The pelvic floor muscle tracking system 1 then tracks the pelvic floor muscles or pelvic floor exercises according to the tracking procedure.

In some examples, the computing device 40 and/or the apparatus 30 manages the tracking procedure and controls it as needed, for example, by determining which exercises are to be performed, by tracking how exercises are being completed, and/or by providing notifications or instructions to the user.

In some examples, the pelvic floor muscle tracking system 1 is configured to determine that the intra-body device 10 has slipped out from the cavity of the person, such as when a slip likelihood has reached a predetermined level, as described elsewhere herein. Such a determination may cause the pelvic floor muscle tracking system 1 to control or adjust the tracking procedure in a specific manner. For example, based on the determination, the pelvic floor muscle tracking system 1 halts tracking of pelvic floor muscles of the person (e.g., until the intra-body device 1 is reinserted), by informing the user (e.g., via a visual and/or audible notification) that the intra-body device 1 has slipped, and/or by instructing (e.g., via a visual and/or audible notification) the user to reintroduce or reinsert the intra-body device 1 into the cavity.

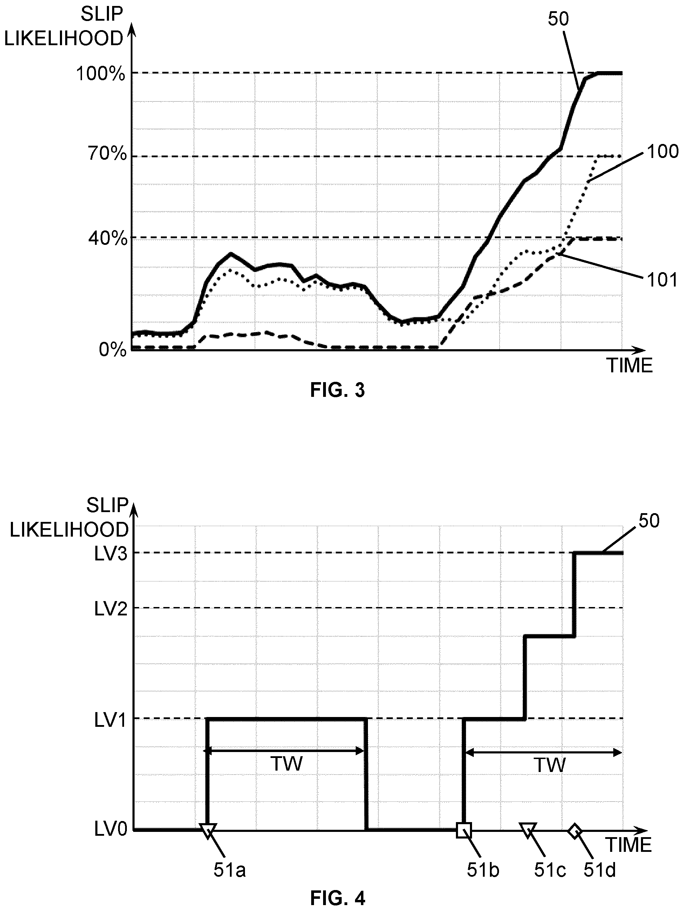

Figure 3 is a graph illustrating examples of a time evolution of slip likelihood 50. The graph shown illustrates a black curve representing the value of the slip likelihood 50 over time, computed according to some examples.

The slip likelihood 50 is established by computing an aggregate of at least one probability 100, 101 of existence of slippage of the concerned intra-body device (e.g., the intra-body device 10). In this example, two sets of processed values are evaluated to compute respective probabilities 100, 101. For example, but without limitation, first processed values for a first probability 100 (shown with a dotted line) are measurements of an accelerometer and second processed values for a second probability 101 (shown with a dashed line) are measurements of a gyroscope.

At least one processor of the intra-body device or of another device receiving data from the intra-body device processes measurements and, thus, the values of the intra-body device over time, especially while a user is performing or is to perform exercises with the intra-body device introduced into a cavity of the body of the user.

At the initial time instant represented, the first probability 100 is above 0% whereas the second probability 101 is 0%. The slip likelihood 50 is above 0% but with a low value, which means that the intra-body device may be within the cavity in the body of the user and well placed therein.

In this example, a first increase in the first probability 100 and, thus, an increase in slip likelihood 50 are computed upon processing at least some values measured by the intra-body device. For example, but without limitation, at least some first values of processed pressure values have an increasing magnitude for at least one frequency above a first predetermined frequency threshold.

After a while, the first probability 100 and the slip likelihood 50 start to decrease owing to more recent measurements that are representative of correct behavior of the intra-body device location-wise.

Then, both the first and second probabilities 100, 101 start to rise at different rates, which may be due to how the values change the probabilities 100, 101 based on predetermined relationships, and/or maximum limits imposed to the rate of change of each probability 100, 101. By way of example, the first probability 100 may be set to maximally change at a rate of 15% per second, and the second probability be set to maximally change at a rate of 10% per second. The maximum limits in rate of change may be set empirically and configured based on the exercise or user, for instance. The use of maximum limits in rate of change is convenient to avoid sudden increases or decreases in probability that could be due to a sporadic incorrect measurement by a sensor.

The increase in the first probability 100 can be due, for example, to at least some processed acceleration values having an increasing magnitude.

As appreciated from the right-most part of the graph of Figure 3, each probability 100, 101 may have a maximum value that cannot be exceeded. In this example, the first probability 100 is limited to 70% and the second probability 101 is limited to 40%. Although the slip likelihood 50 is established by aggregating the first and second probabilities 100, 101, which combined would yield 110% at the right-most part of the graph, the slip likelihood 50 may likewise be maximally limited to 100%.

The determination of the intra-body device having slipped out from the cavity of the user may occur when the slip likelihood reaches or exceeds a certain level, for example, 100% or a value lower than that, e.g., 75%, 85%, etc. In some embodiments, this determination only occurs when two or more probabilities of the at least one probability 100, 101 result from measurements taken during a common time window and the aggregated probability, i.e., the slip likelihood 50, has reached the certain level during that same time window (e.g., several repetitions of an exercise, during performance of a single exercise, etc.). Otherwise, the at least one probability 100, 101 may be reset, for example.

The described first and second probabilities 100, 101 are example acceleration and angular velocity values. It will be noted, however, that other processed values may be used to provide and compute one or more probabilities of existence of slippage, for example but without limitation pressure values including basal pressure values, temperature values, and/or level of power values of captured and/or radiated electromagnetic waves. The computed probabilities may increase or decrease based upon, for example but without limitation, whether:
- processed acceleration values have a magnitude exceeding a predetermined threshold in at least one predetermined acceleration direction and/or have a large component for a frequency above a predetermined frequency threshold;
- processed angular velocity values have a magnitude that exceeds a predetermined threshold;
- processed temperature values have a reduction, lack of change or slow increase over time exceeding a predetermined threshold;
- processed basal pressure values have a decreasing magnitude over time exceeding a predetermined threshold; and/or
- processed pressure values have a magnitude exceeding a predetermined threshold for at least one frequency above a predetermined frequency threshold.

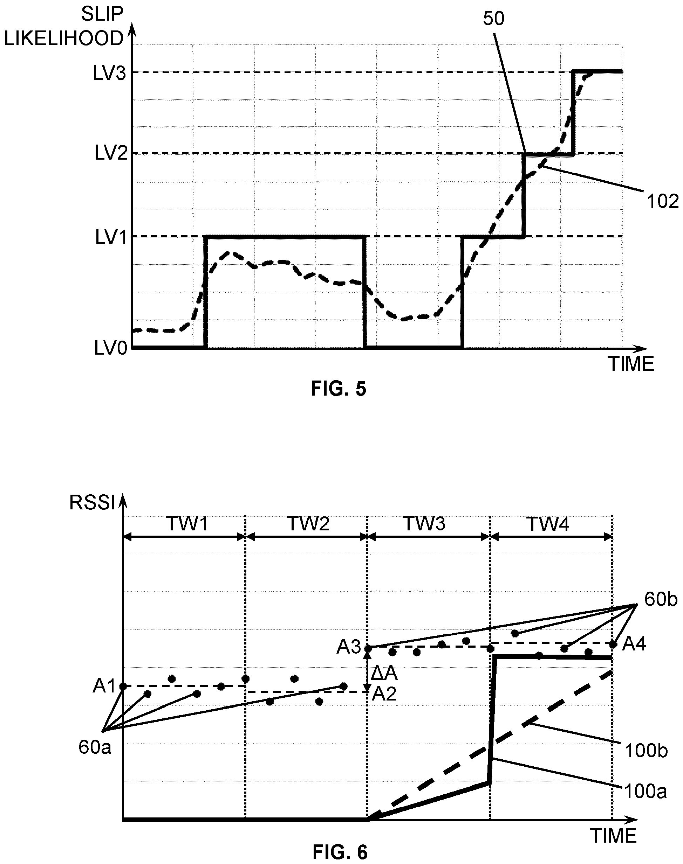

Figure 4 is a graph illustrating examples of a time evolution of slip likelihood 50. The graph shown illustrates a black curve representing the value of the slip likelihood 50 over time, computed according to some examples. Unlike the embodiments described with reference to Figure 3, in the embodiments of Figures 4 and 5 the slip likelihood 50 is established in accordance with predetermined levels. The same behavior of the concerned intra-body device in the example of Figure 3 has also been considered in the examples of Figures 4 and 5.

In Figure 4, the slip likelihood 50 is initially at a predetermined baseline level LV0, which means that the respective intra-body device is within the cavity in the body of the user and which may be indicative of the intra-body device being well placed therein.

At least one processor of the intra-body device or of another device receiving data from the intra-body device processes measurements of the intra-body device over time.

In some occasions, processed measurements of at least one sensor of an intra-body device and/or processed measurements of at least one antenna transmitting and/or receiving wireless data transmissions to/from the intra-body device will be detected as a potential slip situation.

In this example, a first slip situation 51a (illustrated in Figure 4 with a triangle for the sake of the illustration only) is detected upon determining that measurements resulting from the intra-body device meet a condition. For example, but without limitation, a first condition giving rise to the detection of the first slip situation 51a is that at least some first values of processed pressure values have a magnitude that exceeds a first predetermined pressure threshold for at least one frequency above a first predetermined frequency threshold. The slip likelihood 50 is established to a first predetermined level LV1, which means that there is a slip candidate. In this sense, the first predetermined level LV1 may be configured to represent that the intra-body device is moving.

A time window TW is considered for the occurrence of other events that may be detected as potential slip situations. Preferably, the time window TW is set to start at the time of the measurement or measurements (which may not be the time when at least one processor detected the first slip situation 51a) that resulted in the detection of the first slip situation 51a, or to a time previous to it. During the first time window TW, no further slip situations are detected, thus once the time window TW elapses, the slip likelihood 50 is established to the predetermined baseline level LV0.

After returning to the predetermined baseline level LV0, a second slip situation 51b (illustrated with a square in Figure 4 to indicate, for the sake of the illustration only, that it is associated with a condition different than that of the first slip situation 51a) is detected upon determining that measurements resulting from the intra-body device meet a condition.

For example, but without limitation, a second condition giving rise to the detection of the second slip situation 51b is that at least some processed acceleration values have a magnitude that exceeds a predetermined acceleration threshold in at least one predetermined acceleration direction. The slip likelihood 50 is established to the first predetermined level LV1.

Afterwards, within a time window TW starting at the second slip situation 51b, a further slip situation 51c is detected. The third slip situation 51c is the same as the first slip situation 51a in terms of the condition that must be met and, hence, it is also illustrated with a triangle in Figure 4. The third slip situation 51c increases the slip likelihood 50 from the first predetermined level LV1 to the second predetermined level LV2. By the time the third slip situation 51c is detected, it is not necessary that the second slip situation 51b is still detectable. For example, it is not necessary that there is simultaneity in meeting different conditions. In some cases, as soon as one slip condition is detected, that slip condition may not have to be checked for the whole duration of the time window TW again, and it is enough that the slip likelihood 50 is increased to a given level and that the at least one processor keeps track of what slip situations 51a-51d have already been detected in the time window TW to avoid detecting them again.

In some embodiments, the second predetermined level LV2 is configured to represent that the intra-body device has slipped. Hence, the slip likelihood 50 being at the second predetermined level LV2 is enough to determine that a slip has occurred and, thus, trigger any action associated with such an event. In some other embodiments, a greater level of slip likelihood 50 may be required to determine that the slip has occurred, like in the example illustrated, thus the second predetermined level LV2 is configured to represent that there is a slip candidate.

Within the same time window TW, a fourth slip situation 51d is detected that increases the slip likelihood 50 to a predetermined level LV3 greater than LV2. For example, but without limitation, a third condition (illustrated with a 45° rotated square in Figure 4 to indicate, for the sake of the illustration only, that it is associated with a condition different than that of the first, second and third slip situations 51a-51c) is that processed acceleration values are indicative of the variation in acceleration vector exceeding the predetermined acceleration angular variation in a predetermined period of time, or that processed acceleration values have a magnitude that has increased more than the predetermined acceleration increase threshold in the predetermined period of time.

In some examples, the slip likelihood 50 is established to the different predetermined levels LV1-LV3 (and other, if further predetermined levels are set for requirement of further slip situations) in a progressive manner from lower to greater levels. This means that for the slip likelihood 50 to be established to, for instance, the third predetermined level LV3, in such configurations it is a necessary precondition that the slip likelihood is already at the second predetermined level LV2, and that for being established to the second predetermined level LV2 the slip likelihood is already at the first predetermined level LV1. This way, the likelihood of there being a slip increases as more slip situations have been detected.

Even though several slip situations are possible, for establishing the slip likelihood 50, in some embodiments, to the first and/or second predetermined levels LV1, LV2, particular conditions are met, such as, for instance but without limitation, those described in relation to the embodiments of Figure 3 and/or others.

It has been found out that the above two conditions are conditions likely to be met when there is a slip event because the acceleration and pressure values tend to behave in that manner with a lower degree of false positives in comparison with other possible slip situations. However, the present disclosure is not limited in this manner, and other combinations of conditions are possible within the scope of the present disclosure, which also encompasses increasing the slip likelihood 50 irrespective of which condition is met. All or only some conditions may be checked at different times depending on how they are set up.

Figure 5 is a graph illustrating examples of a time evolution of slip likelihood 50.

For establishing the slip likelihood 50, an overall probability of existence of slippage 102 is computed in the same form that the slip likelihood 50 in Figure 3 is computed. For the sake of clarity only, the probability or probabilities considered for the overall probability 102 have not been shown.

A first condition associated with predetermined level LV1 is met when the overall probability 102 or an underlying probability thereof exceeds a predetermined probability value. In this example, the predetermined probability value is 25%.

The slip likelihood 50 may return to a baseline level LV0 when the first condition is not met and/or after some time has elapsed after the slip likelihood 50 was established to the predetermined level LV1.

Later, subsequent condition(s) are met that are associated with further predetermined levels. A second condition is associated with predetermined level LV2 and is met when the overall probability 102 or an underlying probability thereof exceeds a predetermined probability value, thereby increasing the slip likelihood 50 accordingly. In this example, the predetermined probability value is 60%. In this case, there is also a third condition associated with predetermined level LV3 and is met when the overall probability 102 or an underlying probability thereof exceeds a predetermined probability value, thereby increasing the slip likelihood 50 accordingly. In this example, the predetermined probability value is 90%.

In some embodiments, when the slip likelihood 50 is established, for example, at predetermined level LV1 or at predetermined level LV2, it may still be determined that the intra-body device has not slipped out from the cavity of the user, yet there is a potential slip situation. One or more commands may be provided in the situations such as, for example, notify the user by providing one or more user perceptible signals of a potential slip situation being underway (e.g., as described with reference to Figure 2). The user may take corrective action to avoid intra-body device's slippage.

In some embodiments, a single condition and a single predetermined level are set whereas, in some other embodiments, multiple conditions and/or multiple predetermined levels are set.

Figure 6 is a graph illustrating examples of a potential slip situation. The graph shows RSSI values 60a, 60b received at a device remote from an intra-body device versus time. The device can, for example, the apparatus 40 or the computing device 30 of Figure 2.

The RSSI values 60a, 60b, which could be replaced with direct measurements of the antenna(s) of the receiving end of wireless data communications where the intra-body device is the transmitter, for example, are indicative of a potential slip situation.

During first time windows TW1, TW2, the RSSI values 60a have a given level that generally oscillates around average values A1, A2, respectively. During second time windows TW3, TW4, the RSSI values 60b have a given level that generally oscillates around average values A3, A4, respectively. Different time windows (which may be shorter than a common predetermined time window TW) may be provided to consider a number of RSSI values 60a, 60b for averaging them all and obtain a respective average value.

A difference ΔA between two average values, which may be consecutive or not, is, in some examples, to be compared with a predetermined power level threshold. When the difference exceeds ΔA such threshold, for example between the average value A2 of the last time window TW2 of the first time windows and the average value A3 of the first time window TW3 of the second time windows, a slip situation may exist and be, at least partially, detected. Such considerable different values of average RSSI or level of power can be associated with the intra-body device exiting the cavity and having better wireless transmission capabilities with respect to the device at the receiving end.

An example probability 100a of existence of slippage of the intra-body device has been represented in the graph with a solid line, as computed according to some examples. The sudden rise in RSSI values 60b in the second time window TW3 and non-reduction thereof to the levels of RSSI values 60a of the first time windows TW1, TW2 increases the probability 100a of existence of slippage, for example from 0% to 5% if percentage values are used. The difference between the average value A3 and the average value A2 once the average value A3 is obtained further increases the probability 100a, for example from 5% to 25% if percentage values are used.

Another example probability 100b of existence of slippage of the intra-body device has been represented in the graph with a dashed line. The probability 100b is computed such that changes in the RSSI values 60b with respect to previous RSSI values 60a increase or reduce the probability 100b with time depending on how much the values change and whether the magnitude of the RSSI values increases or decreases. In this case, in the second time windows TW3, TW4 the probability 100b keeps increasing as there is no reduction of the RSSI values 60b to go back to levels such as those of the first time windows TW1, TW2.

Computation of the probability 100a, 100b in one way or another may be chosen depending on the which values are being processed (e.g., measurements of a particular sensor, RSSI values, etc.), and/or who is the user using the intra-body device, and/or the exercise performed by the user.

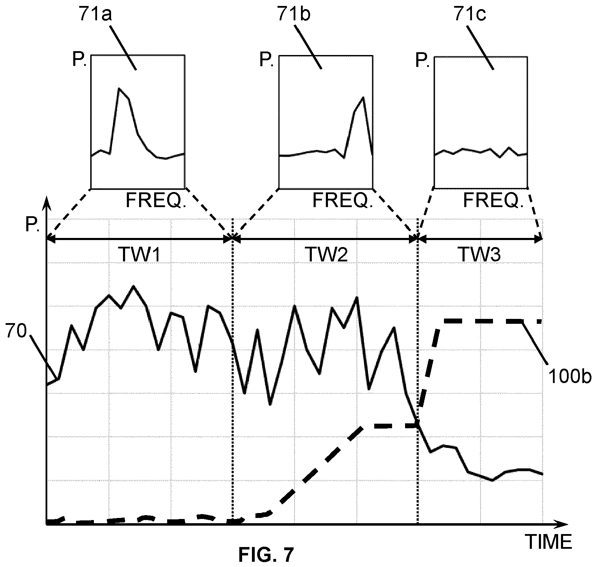

Figure 7 is a graph illustrating examples of potential slip situations. The graph shows pressure values 70 measured by an intra-body device versus time, and frequency spectra 71a-71c of the pressure values 70 for the measurements occurring during respective time windows TW1-TW3.

The pressure values 70, which are provided by the intra-body device for enabling the tracking of the pelvic floor muscles (e.g., by the intra-body device 10 to the computing device 30 of Figure 2), reflect the force or pressure exerted by the pelvic floor muscles on the pressure sensor of the intra-body device. Their behavior is also indicative of the status of the intra-body device in what regards its insertion, or not, within the cavity.

In a first time window TW1, the frequency spectrum 71a obtained from, e.g., applying a discrete Fourier transform to the pressure values measured within that time window TW1, shows a peaks at mid-range frequencies, for example, between 0,5 Hz and 2,5 Hz. This is the behavior generally observed when the intra-body device is inserted in the cavity and the person exercises normally.

In a second time window TW2 posterior to the first time window TW1, the frequency spectrum 71b is obtained in the same fashion for measurements taken during that time window TW2 shows one or more peaks at high frequencies. This is the behavior generally observed when the intra-body device is exiting the cavity. This can be associated with a first slip situation having occurred, especially after having observed that in the first time window TW1 there was one or more peaks at mid-range frequencies. When computing a probability 100b of existence of slippage, the behavior in the second time window TW2 after the behavior in the first time window TW1 results in an increase in the probability 100b, for example a progressive increase with a limit, which may be a local or a global maximum for the probability 100b. In this example, the limit is a local limit.

Further, in a third time window TW3 posterior to both the first and second time windows TW1, TW2, the frequency spectrum 71c obtained in the same fashion for measurements taken during that time window TW3 does not show any peaks for any frequency. This is the behavior generally observed when the intra-body device is outside the cavity and, thus, the device is not subjected to any significant force or pressures that the pressure sensor could measure. This can be associated with a second slip situation having occurred, especially after having observed the behavior in the first and second time windows TW1, TW2. When computing a probability 100b of existence of slippage, the behavior in the third time window TW3 after the behavior in the second time window TW2 results in an additional increase in the probability 100b with a greater rate than the increase in the second time window TW2. This is so because the behavior is even more significant than that of the second time window TW2. The increase in probability 100b may be limited by a top limit, in this case a maximum limit.

It is in any case noted that, in other embodiments, a pressure behavior like that of the third time window TW3 may take place even without the behavior of the second time window TW2. For instance, the intra-body device may slip from the cavity progressively without many changes in the pressure that would show peaks at higher frequencies.

In order to analyze the relevant portions of the frequency spectra 71a-71c to compute a probability 100b of existence of slippage and/or determine whether slip situations may have occurred, different techniques are possible. For example, a high-pass filter that removes lower frequencies may be applied to the measurements or the spectrum to only process the amplitudes of the pressure values for upper frequencies, e.g., of 3 Hz and above. Or, by way of another example, a low-pass filter that removes high frequencies may be applied to the measurements or the spectrum to only process the amplitudes of the pressure values for low frequencies, e.g., of 1 Hz and below.

Since in the slip situations described with reference to Figures 6 and 7, multiple measurements are considered for each time window, for the sake of determining whether any of such slip situations occurred during a common predetermined time window already triggered by another slip situation, as described in reference to Figure 4, any time instant of the respective time window TW1-TW4 can be considered. Preferably, but not necessarily, the first time instant of the relevant time window TW1-TW4 is considered as the occurrence of the slip situation. Therefore, in the case of the slip situation of Figure 6, the time instant associated with the detected slip situation is the first of the first time window TW3 of the second time windows whereas, in the case of the slip situations of Figure 7, the time instants associated with the detected slip situations is the first of the second and third time windows, TW2, TW3, respectively. However, as aforesaid, this can be configured differently in other embodiments without departing from the scope of the present disclosure.

In this text, the terms "first", "second", or the like have been used herein to describe several elements, devices or parameters, it will be understood that the elements, devices or parameters should not be limited by these terms since the terms are only used to distinguish one element, device or parameter from another.

In this text, the term "includes", "comprises" and derivations thereof (such as "including", "comprising", etc.) should not be understood in an excluding sense, that is, these terms should not be interpreted as excluding the possibility that what is described and defined may include further elements, steps, etc.

On the other hand, the disclosure is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

Although specific examples are described herein, it will be evident that various modifications and changes may be made to these examples without departing from the broader spirit and scope of the disclosure. Accordingly, the specification and drawings are to be regarded in an illustrative rather than a restrictive sense. The accompanying drawings that form a part hereof show by way of illustration, and not of limitation, specific examples in which the subject matter may be practiced. The examples illustrated are described in sufficient detail to enable those skilled in the art to practice the teachings disclosed herein. Other examples may be utilized and derived therefrom, such that structural and logical substitutions and changes may be made without departing from the scope of this disclosure. This detailed description, therefore, is not to be taken in a limiting sense, and the scope of various examples is defined only by the appended claims, along with the full range of equivalents to which such claims are entitled.

Such examples of the subject matter may be referred to herein, individually or collectively, by the term "embodiment" or "example" merely for convenience and without intending to voluntarily limit the scope of this application to any single example or concept if more than one is in fact disclosed. Thus, although specific examples have been illustrated and described herein, it should be appreciated that any arrangement calculated to achieve the same purpose may be substituted for the specific examples shown. This disclosure is intended to cover any and all adaptations or variations of various examples. Combinations of the above embodiments or examples, and other examples not specifically described herein, will be apparent to those of skill in the art upon reviewing the above description.

Some portions of the subject matter discussed herein may be presented in terms of algorithms or symbolic representations of operations on data stored as bits or binary digital signals within a machine memory (e.g., a computer memory). Such algorithms or symbolic representations are examples of techniques used by those of ordinary skill in the data processing arts to convey the substance of their work to others skilled in the art. As used herein, an "algorithm" is a self-consistent sequence of operations or similar processing leading to a desired result. In this context, algorithms and operations involve physical manipulation of physical quantities. Typically, but not necessarily, such quantities may take the form of electrical, magnetic, or optical signals capable of being stored, accessed, transferred, combined, compared, or otherwise manipulated by a machine. It is convenient at times, principally for reasons of common usage, to refer to such signals using words such as "data," "content," "bits," "values," "elements," "symbols," "characters," "terms," "numbers," "numerals," or the like. These words, however, are merely convenient labels and are to be associated with appropriate physical quantities.

Unless specifically stated otherwise, discussions herein using words such as "processing," "computing," "calculating," "determining," "presenting," "displaying," or the like may refer to actions or processes of a machine (e.g., a computer) that manipulates or transforms data represented as physical (e.g., electronic, magnetic, or optical) quantities within one or more memories (e.g., volatile memory, non-volatile memory, or any suitable combination thereof), registers, or other machine components that receive, store, transmit, or display information. Furthermore, unless specifically stated otherwise, the terms "a" and "an" are herein used, as is common in patent documents, to include one or more than one instance. As used herein, the conjunction "or" refers to a non-exclusive "or," unless specifically stated otherwise.

Unless the context clearly requires otherwise, throughout the description and the claims, the words "comprise," "comprising," and the like are to be construed in an inclusive sense, as opposed to an exclusive or exhaustive sense, e.g., in the sense of "including, but not limited to." As used herein, the terms "connected," "coupled," or any variant thereof means any connection or coupling, either direct or indirect, between two or more elements; the coupling or connection between the elements can be physical, logical, or a combination thereof. Additionally, the words "herein," "above," "below," and words of similar import, when used in this application, refer to this application as a whole and not to any particular portions of this application. Where the context permits, words using the singular or plural number may also include the plural or singular number, respectively. The word "or" in reference to a list of two or more items, covers all of the following interpretations of the word: any one of the items in the list, all of the items in the list, and any combination of the items in the list.

Although some examples may include a particular sequence of operations, the sequence may in some cases be altered without departing from the scope of the present disclosure. For example, some of the operations may be performed in parallel or in a different sequence that does not materially affect the functions as described in the examples. In other examples, different components of an example device or system that implements an example method may perform functions at substantially the same time or in a specific sequence.

As used herein, the term "processor" may refer to any one or more circuits or virtual circuits (e.g., a physical circuit emulated by logic executing on an actual processor) that manipulates data values according to control signals (e.g., commands, opcodes, machine code, control words, macroinstructions, etc.) and which produces corresponding output signals that are applied to operate a machine. A processor may, for example, include at least one of a Central Processing Unit (CPU), a Reduced Instruction Set Computing (RISC) Processor, a Complex Instruction Set Computing (CISC) Processor, a Graphics Processing Unit (GPU), a Digital Signal Processor (DSP), a Tensor Processing Unit (TPU), a Neural Processing Unit (NPU), a Vision Processing Unit (VPU), a Machine Learning Accelerator, an Artificial Intelligence Accelerator, an Application Specific Integrated Circuit (ASIC), a Field Programmable Gate Array (FPGA), a Radio-Frequency Integrated Circuit (RFIC), a Neuromorphic Processor, a Quantum Processor, or any combination thereof. A processor may be a multi-core processor having two or more independent processors (sometimes referred to as "cores") that may execute instructions contemporaneously. Multi-core processors may contain multiple computational cores on a single integrated circuit die, each of which can independently execute program instructions in parallel. Parallel processing on multi-core processors may be implemented via architectures like superscalar, VLIW, vector processing, or SIMD that allow each core to run separate instruction streams concurrently. A processor may be emulated in software, running on a physical processor, as a virtual processor or virtual circuit. The virtual processor may behave like an independent processor but is implemented in software rather than hardware.

The various operations of example methods described herein may be performed, at least partially, by one or more processors that are temporarily configured (e.g., by software) or permanently configured to perform the relevant operations. Whether temporarily or permanently configured, such processors may constitute processor-implemented modules/components that operate to perform one or more operations or functions. The modules/components referred to herein may, in some examples, comprise processor-implemented modules/components.

Similarly, the methods described herein may be at least partially processor-implemented. For example, at least some of the operations of a method may be performed by one or more processors or processor-implemented modules/components. The performance of certain of the operations may be distributed among the one or more processors, not only residing within a single machine, but deployed across a number of machines. In some examples, the processor or processors may be located in a single location (e.g., within a home environment, an office environment, or a server farm), while in other examples the processors may be distributed across a number of locations.

Examples may be implemented in digital electronic circuitry, or in computer hardware, firmware, or software, or in combinations of them. Examples may be implemented using a computer program product, such as a computer program tangibly embodied in an information carrier, such as in a machine-readable medium for execution by, or to control the operation of, data processing apparatus, such as a programmable processor, a computer, or multiple computers.

A computer program can be written in any form of programming language, including compiled or interpreted languages, and it can be deployed in any form, including as a standalone program or as a module, subroutine, or other unit suitable for use in a computing environment. A computer program can be deployed to be executed on one computer or on multiple computers at one site or distributed across multiple sites and interconnected by a communication network.

In view of the above-described implementations of subject matter this application discloses the following list of non-limiting examples, wherein one feature of an example in isolation or more than one feature of an example, taken in combination and, optionally, in combination with one or more features of one or more further examples are further examples also falling within the disclosure of this application.

Example 1 is a system comprising: at least one processor; and memory storing instructions that, when executed by the at least one processor, configure the at least one processor to perform operations comprising: processing values comprising at least one of measurements by one or more sensors of an intra-body device, power levels of wireless data transmissions transmitted by one or more antennas of the intra-body device, or power levels of wireless data transmissions received by the one or more antennas of the intra-body device; establishing a slip likelihood based on the processed values, the slip likelihood being a likelihood of the intra-body device having slipped out from a cavity of a person; detecting that the slip likelihood has reached or exceeded a predetermined level; in response to detecting that the slip likelihood has reached or exceeded the predetermined level, identifying that the intra-body device has slipped out from the cavity of the person; and, optionally, controlling a tracking procedure of the system based on identifying that the intra-body device has slipped out from the cavity of the person.

In Example 2, the subject matter of Example 1 includes, wherein the system comprises a pelvic floor muscle tracking system, and the cavity is a vagina or an anus of the person.

In Example 3, the subject matter of Example 2 includes, wherein the one or more sensors of the intra-body device comprise a pressure sensor, and controlling the tracking procedure of the system based on identifying that the intra-body device has slipped out from the cavity of the person comprises halting tracking of pelvic floor muscles of the person based on processed pressure values of the pressure sensor.

In Example 4, the subject matter of Examples 1-3 includes, wherein controlling the tracking procedure of the system based on identifying that the intra-body device has slipped out from the cavity of the person comprises causing a notification to be provided to the person, the notification comprising at least one of an indication that the intra-body device has slipped out from the cavity or an instruction to reintroduce the intra-body device into the cavity.

In Example 5, the subject matter of Examples 1-4 includes, wherein establishing the slip likelihood comprises computing the slip likelihood based on at least one probability of existence of slippage.

In Example 6, the subject matter of Example 5 includes, wherein establishing the slip likelihood comprises associating at least one predetermined relationship between some or all processed values and the at least one probability of existence of slippage.

In Example 7, the subject matter of Examples 5-6 includes, wherein the at least one probability of existence of slippage comprises a plurality of probabilities, and establishing the slip likelihood comprises aggregating some or all of the plurality of probabilities.

In Example 8, the subject matter of Examples 5-7 includes, wherein establishing the slip likelihood comprises weighing one or more probabilities of the at least one probability of existence of slippage.

In Example 9, the subject matter of Examples 5-8 includes, wherein establishing the slip likelihood comprises adjusting a maximum limit for one or more probabilities of the at least one probability of existence of slippage based on at least one of an exercise to be performed by the person or the person to perform the exercise.

In Example 10, the subject matter of Examples 5-9 includes, wherein the one or more sensors of the intra-body device comprise a motion sensor, wherein a probability of the at least one probability of existence of slippage relates to processed angular velocity values of the motion sensor, and the probability is increased in response to detecting that at least some of the processed angular velocity values have at least one of an increasing magnitude over time or a magnitude exceeding a predetermined angular velocity threshold.

In Example 11, the subject matter of Examples 5-10 includes, wherein the one or more sensors of the intra-body device comprise a pressure sensor, wherein a probability of the at least one probability of existence of slippage relates to processed pressure values of the pressure sensor, and the probability is increased in response to detecting at least one of: at least some of the processed pressure values have at least one of an increasing magnitude over time or a magnitude outside of a predetermined pressure range for at least one frequency above a first predetermined frequency threshold; or at least some of the processed pressure values have at least one of a decreasing magnitude over time or a magnitude outside a predetermined pressure range for at least one frequency above a second predetermined frequency threshold and below the first predetermined frequency threshold.

In Example 12, the subject matter of Examples 5-11 includes, wherein the one or more sensors of the intra-body device comprise an acceleration sensor, wherein a probability of the at least one probability of existence of slippage relates to processed acceleration values of the acceleration sensor, and the probability is increased in response to detecting that at least some of the processed acceleration values have at least one of an increasing magnitude over time or a magnitude within a predetermined acceleration range in at least one predetermined acceleration direction.

In Example 13, the subject matter of Examples 5-12 includes, wherein the one or more sensors comprise a temperature sensor, wherein a probability of the at least one probability of existence of slippage relates to processed temperature values of the temperature sensor, and the probability is increased in response to detecting that at least some of the processed temperature values are indicative of a predetermined rate of temperature change.

In Example 14, the subject matter of Example 13 includes, wherein the predetermined rate of temperature change comprises at least one of a predetermined rate of temperature invariance over time, a temperature decrease over time, or a slow rate of temperature increase over time.

In Example 15, the subject matter of Examples 1-14 includes, wherein the one or more sensors of the intra-body device comprise a pressure sensor, and the operations further comprise, prior to detecting that the slip likelihood has reached or exceeded the predetermined level and while the intra-body device is introduced into a vagina or an anus of the person: tracking pelvic floor muscles of the person to establish how the pelvic floor muscles are contracting or elongating based on processed pressure values of the pressure sensor.

In Example 16, the subject matter of Examples 1-15 includes, the operations further comprising, prior to detecting that the slip likelihood has reached or exceeded the predetermined level, causing a notification to be provided to the person, the notification comprising an indication that the intra-body device will potentially slip out from the cavity.

In Example 17, the subject matter of Examples 1-16 includes, wherein the predetermined level is a second predetermined level that is greater than a first predetermined level, wherein the one or more sensors of the intra-body device comprise a motion sensor and a pressure sensor, and wherein the processed values comprise processed acceleration values measured by the motion sensor and processed pressure values measured by the pressure sensor, the operations further comprising: establishing the slip likelihood to the first predetermined level in response to detecting that one of a first condition or a second condition is met; and establishing the slip likelihood to the second predetermined level in response to detecting that the slip likelihood is at the first predetermined level and the first condition and the second condition are both met, wherein the first condition requires that at least some of the processed acceleration values have a magnitude in at least one predetermined acceleration direction that exceeds a predetermined acceleration threshold, and wherein the second condition requires that at least some first values of the processed pressure values have a magnitude that exceeds a first predetermined pressure threshold for at least one frequency above a first predetermined frequency threshold, wherein identifying that the intra-body device has slipped out from the cavity of the person is in response to establishing the slip likelihood to the second predetermined level, and wherein the processed acceleration values and the processed pressure values considered for establishing the slip likelihood to the first predetermined level and the second predetermined level are measured during a common time window.

Example 18 is an intra-body device comprising: one or more sensors; at least one processor; and memory storing instructions that, when executed by the at least one processor, configure the at least one processor to perform operations comprising: processing values comprising at least one of measurements by the one or more sensors, power levels of wireless data transmissions transmitted by one or more antennas of the intra-body device, or power levels of wireless data transmissions received by the one or more antennas of the intra-body device; establishing a slip likelihood based on the processed values, the slip likelihood being a likelihood of the intra-body device having slipped out from a cavity of a person; detecting that the slip likelihood has reached or exceeded a predetermined level; in response to detecting that the slip likelihood has reached or exceeded the predetermined level, identifying that the intra-body device has slipped out from the cavity of the person; and causing a tracking procedure to be controlled based on identifying that the intra-body device has slipped out from the cavity of the person.

In Example 19, the subject matter of Example 18 includes, wherein the one or more sensors comprise a pressure sensor, and the cavity is a vagina or an anus of the person.

Example 20 is a method comprising: processing, by at least one processor, values comprising at least one of measurements by one or more sensors of an intra-body device, power levels of wireless data transmissions transmitted by one or more antennas of the intra-body device, or power levels of wireless data transmissions received by the one or more antennas of the intra-body device; establishing, by the at least one processor, a slip likelihood based on the processed values, the slip likelihood being a likelihood of the intra-body device having slipped out from a cavity of a person; detecting, by the at least one processor, that the slip likelihood has reached or exceeded a predetermined level; in response to detecting that the slip likelihood has reached or exceeded the predetermined level, identifying, by the at least one processor, that the intra-body device has slipped out from the cavity of the person; and controlling, by the at least one processor, a tracking procedure based on identifying that the intra-body device has slipped out from the cavity of the person.

Example 21 is at least one machine-readable medium including instructions that, when executed by processing circuitry, cause the processing circuitry to perform operations to implement any of Examples 1-20.

Example 22 is an apparatus comprising means to implement any of Examples 1-20.

Example 23 is a system to implement any of Examples 1-20.

Example 24 is a method to implement any of Examples 1-20.

## Claims

1. A method comprising:
digitally processing values measured by one or more sensors of an intra-body device, and/or values of level of power of wireless data transmissions transmitted and/or received by one or more antennas of the intra-body device;
digitally establishing a slip likelihood based on the processed values, wherein the slip likelihood is a likelihood of the intra-body device having slipped out from a cavity of a person; and
digitally determining that the intra-body device has slipped out from the cavity of the person when the slip likelihood has at least reached a predetermined level.

2. The method of claim 1, further comprising, when it is determined that the intra-body device has slipped out from the cavity of the person, halting tracking of pelvic floor muscles of the person based on processed pressure values of a pressure sensor of the intra-body device.

3. The method of any one of the preceding claims, wherein establishing the slip likelihood comprises computing the slip likelihood based on at least one probability of existence of slippage.

4. The method of claim 3, wherein establishing the slip likelihood comprises associating at least one predetermined relationship between some or all processed values and the at least one probability of existence of slippage.

5. The method of any one of claims 3-4, wherein establishing the slip likelihood comprises aggregating some or all probabilities of the at least one probability of existence of slippage.

6. The method of any one of claims 3-5, wherein establishing the slip likelihood comprises weighing one or more probabilities of the at least one probability of existence of slippage and/or adjusting a maximum limit for the one or more probabilities based on an exercise to be performed by a user and/or based on the user to perform the exercise.

7. The method of any one of claims 3-6, wherein a probability of the at least one probability of existence of slippage relates to processed angular velocity values of a motion sensor of the intra-body device and increases when at least some values of the processed angular velocity values have an increasing magnitude over time and/or the magnitude exceeds a predetermined angular velocity threshold.

8. The method of any one of claims 3-7, wherein a probability of the at least one probability of existence of slippage relates to processed pressure values of a pressure sensor of the intra-body device and increases when:
at least some values of the processed pressure values have an increasing magnitude over time and/or a magnitude outside a predetermined pressure range for at least one frequency above a first predetermined frequency threshold; and/or
at least some values of the processed pressure values have a decreasing magnitude over time and/or a magnitude outside a predetermined pressure range for at least one frequency above a second predetermined frequency threshold and below the first predetermined frequency threshold.

9. The method of any one of claims 3-8, wherein a probability of the at least one probability of existence of slippage relates to processed acceleration values of an acceleration sensor of the intra-body device and increases when at least some processed acceleration values have an increasing magnitude over time and/or a magnitude within a predetermined acceleration range in at least one predetermined acceleration direction.

10. The method of any one of the preceding claims, wherein a probability of the at least one probability of existence of slippage relates to processed temperature values of a temperature sensor of the intra-body device and increases when at least some values of processed temperature values are indicative of a rate of temperature invariancy or temperature decrease or a slow rate of temperature increase over time; said probability at least reaching the predetermined level when the at least some values of processed temperature values are indicative of the rate of temperature invariancy or temperature decrease or the slow rate of temperature increase over time.

11. The method of any one of the preceding claims, further comprising, when the slip likelihood is below the predetermined level, digitally tracking pelvic floor muscles to establish how muscles of a pelvic floor of the user are contracting or elongating based on processed pressure values of a pressure sensor of the intra-body device.

12. The method of any one of the preceding claims, further comprising, prior to determining that the intra-body device has slipped out from the cavity of the person, digitally commanding to provide or providing one or more user perceptible signals indicative of the intra-body device potentially slipping out from the cavity.

13. The method of any one of the preceding claims, wherein:
the processed values at least comprise acceleration values measured by a motion sensor of the intra-body device and pressure values measured by a pressure sensor of the intra-body device;
the slip likelihood is established to a first predetermined level when one of first and second conditions is met, and to a second predetermined level when the slip likelihood is in the first predetermined level and the first and second conditions are both met, wherein the first condition requires that at least some processed acceleration values have a magnitude in at least one predetermined acceleration direction that exceeds a predetermined acceleration threshold, and wherein the second condition requires that at least some first values of the processed pressure values have a magnitude that exceeds a first predetermined pressure threshold for at least one frequency above a first predetermined frequency threshold;
determining that the intra-body device has slipped out from the cavity of the person when the slip likelihood has at least reached the second predetermined level, the second predetermined level being greater than the first predetermined level; and
the processed acceleration and pressure values considered for establishing the slip likelihood to the first and second predetermined levels are measured during a common predetermined time window.

14. A computing device or system comprising means adapted to execute the steps of a method according to any one of the preceding claims.

15. An intra-body device comprising:
at least one pressure sensor;
at least one motion sensor; and
at least one computing device or system according to claim 14.
